# EUROPÄISCHE PATENTSCHRIFT

(11) **EP 3 463 497 B1**
(45) Veröffentlichungstag und Bekanntmachung des Hinweises auf die Patenterteilung: **16.11.2022**
(21) Anmeldenummer: 17721710.6
(22) Anmeldetag: 08.05.2017
(51) Int. Cl.: A61L 27/16, A61L 27/56, A61F 2/30

(54) **IMPLANTATHERSTELLVERFAHREN MITTELS ADDITIVEM SELEKTIVEM LASERSINTERN UND IMPLANTAT**
IMPLANT PRODUCTION METHOD USING ADDITIVE SELECTIVE LASER SINTERING, AND IMPLANT
PROCÉDÉ DE FABRICATION ADDITIVE D'IMPLANT PAR FRITTAGE SÉLECTIF PAR LASER ET IMPLANT

(30) Priorität: 07.06.2016 DE 102016110500
(43) Veröffentlichungstag der Anmeldung: 10.04.2019
(73) Patentinhaber: Karl Leibinger Medizintechnik GmbH & Co. KG, 78570 Mühlheim (DE)
(72) Erfinder: AKSU, Adem, 78054 Villingen-Schwenningen (DE); REINAUER, Frank, 78576 Emmingen-Liptingen (DE); WOLFRAM, Tobias, 63303 Dreieich (DE)
(74) Vertreter: Winter, Brandl - Partnerschaft mbB
(86) Internationale Anmeldenummer: PCT/EP2017/060903
(87) Internationale Veröffentlichungsnummer: WO 2017/211522

(56) Entgegenhaltungen:
- WO-A1-2009/014718
- DE-A1- 10 055 465
- CHANGHUI SONG ET AL: "Customized UHMWPE tibial insert directly fabricated by selective laser sintering", INTERNATIONAL JOURNAL OF ADVANCED MANUFACTURING TECHNOLOGY, SPRINGER VERLAG, LONDON; GB, Bd. 85, Nr. 5, 4. November 2015 (2015-11-04), Seiten 1217-1226, XP035988750, ISSN: 0268-3768, DOI: 10.1007/S00170-015-8046-6 [gefunden am 2015-11-04]

## Beschreibung

Die Erfindung betrifft ein Verfahren zum Herstellen eines Implantats, wobei schon bekannt ist, bestimmte Partikel, insbesondere UHMWPE-Partikel (Ultra-high-molecular-weight Polyethylene - Partikel) zu verarbeiten. Hier wird unter UHMWPE eine gereinigte, synthetisch reine Form der Partikel verstanden.

So offenbart beispielsweise die US 6,641,617 B1 eine strahlungsbehandelte medizinische Prothese aus UHMWPE. Dabei wird ein Schmelzen des UHMWPE durchgeführt wobei im Wesentlichen keine nachweisbaren freien Radikale vorhanden sind.

Die EP 1 563 857 A2 offenbart ferner ein Verfahren zum Herstellen von abtragungs- und oxidationsresistentem Polyethylen (PE). Dabei wird Polyethylen bei einer Temperatur unterhalb dessen Schmelztemperatur bereitgestellt und dann bestrahlt, um eine Vernetzung zu erreichen und um ausreichend Wärme zu erzeugen sowie um das Polyethylen zumindest teilweise zu schmelzen. Danach wird das Polyethylen abgekühlt.

Die US 8,142,886 B2 und die WO 2009/014718 A1 offenbaren eine laser-gesinterte, poröse Polymervorrichtung, die einen Kern mit einer gewissen Menge an anorganischem Material aufweist. Der Kern weist zumindest zwei weitere Schichten auf, wobei das anorganische Material eine Mischung aus mindestens zwei Komponenten der Gruppe Metall / Metalllegierung, Calciumphosphat, Edelstahl und Glas umfasst.

Aus der EP 1 276 436 A1 ist auch ein Implantat zu einem Verfahren zur Verbesserung der Verschleißbeständigkeit und Oxidationsbeständigkeit eines Implantates bekannt, wobei UHMWPE verwendet wird und ein Bestrahlen des Implantats oberhalb von vier Mrad (Die Definition für die Einheit Mrad lautet: 1 Rad = 0,01 Gy = 0,01 J/kg) durchgeführt wird. Ferner wird dort das Einmischen eines Oxidationsmittels mit Polyethylenpulver offenbart.

Aus der US 2014/0052264 A1 ist auch ein poröses Implantat mit einer Vielzahl von gesinterten Polymerpartikeln bekannt, wobei ein Antioxidantium auf der Oberfläche vorhanden ist. Im Kern dieser Patentanmeldung steht also ein poröses Implantat mit einer Vielzahl von Polymerpartikeln, die derart gesintert sind, dass sie ein an mehreren Kontaktstellen verbundenes poröses Geflecht mit Poren bilden, wobei die Vielzahl von Polymerpartikeln auch Polyethylen beinhalten kann. Das Antioxidantium wird auf einer Oberfläche wenigstens einiger Polymerpartikel und/oder in den Poren des porösen Geflechts angeordnet.

Aus den Publikationen "Selective Laser Sintering of Ultra High Molecular Weight Polyethylene for Clinical Application" von J. Rimell et. al. in J Biomed Mater Res (ApplBiometer) 53, 200, S. 414-420 sowie "Polymer Poweders for Selecetive Laser Sintering (SLS)" von M. Schmid et. al. in AIP Conf. Proc. 1664 160009 (2015) sind ferner Verfahren zur Herstellung eines Implantats bekannt.

Die DE 100 55 465 A1 offenbart ein Knochenersatzwerkstoff sowie ein Verfahren zur Herstellung eines Knochenersatz-Implantats. Der Knochenersatzwerkstoff weist dabei einen Matrixwerkstoff aus einem biokompatiblen lasersintbaren Polymermaterial und in den Matrixwerkstoff zumindest teilweise eingebettete Füllstoffpartikel aus anorganischen, nichtmetallischen, insbesondere bioinerten oder bioaktiven, Materialien. Das Verfahren ist durch die Verfahrensschritte eines Bereitstellens eines pulverförmigen Gemischs oder Compound-Materials eines lasersintbaren Matrix-Polymermaterials, eines schichtweisen Anordnens des Ausgangsmaterials in einer Pulverschicht, einem Lasersintern einer Lage und eines sukzessiven Wiederholens bestimmt.

Es ist die Aufgabe der vorliegenden Erfindung, ein schnelleres, kostengünstigeres und einfacher durchzuführendes Verfahren zur Verfügung zu stellen, das zu Implantaten führt, die schneller und erfolgreicher in das Gewebe eines Säugers integriert werden können.

Diese Aufgabe wird erfindungsgemäß dadurch gelöst, dass beispielsweise ausschließlich Partikel der Gruppe aus Ultra-high-molecular-weight Polyethylene (UHMWPE) und/oder High-density Polyethylene (HDPE) und/oder Polyprophylen (PP), insbesondere auch daraus bestehende aber artunterschiedliche Mischungen, mittels eines Selective Laser Sintering Verfahrens (SLS-Verfahrens) schichtweise miteinander verschmolzen werden und eine Wärmebehandlung nach dem Selective Lasersintern so durchgeführt wird, dass die Poren des zu schaffenden Implantats unverschlossen bleiben oder eine Wärmebehandlung nach dem Selective Lasersintern so durchgeführt wird, dass die Poren des zu schaffenden Implantats nur in Teilbereichen geschlossen werden. Dadurch wird die Stabilität verbessert und das Einwachsen wird weiter auf gutem Niveau ermöglicht. Es können auch noch weitere, beispielsweise als Füllstoffe agierende Partikel beigemengt werden. Es lassen sich also UHMWPE, HDPE und PP jeweils in Reinform nur für sich verwenden oder in zweier Mischungsverhältnissen oder in einer Mischung aus allen drei Partikelarten. Als Zusatzstoffe bzw. Beimischungen bieten sich Materialien wie z.B. HAP, CaCO3, Mg, alpha/beta TCP oder anderen Polyester-Materialien wie z.B. PDLLA, PLGA, PLA, PGA, Chitosan-Fasern, Chitosan-Partikel an.

Gerade die Komponenten UHMWPE, HDPE und PP haben sich bei der Verwendung von der Herstellung von Implantaten bewährt. Diese Implantate zeigen zumindest partiell ein gewünschtes Einwachsen von Weichgewebe und Knochengewebe. Selbst erste, einer Geheimhaltung unterliegenden klinische Versuche sind erfolgreich, insbesondere bei entsprechender Strukturierung der neuen Implantate. Hier zeigt sich dann ein besonders gutes Einwachsen.

Vorteilhafte Ausführungsformen sind in den Unteransprüchen beansprucht und werden nachfolgend näher erläutert.

Es ist von Vorteil, wenn die Partikel zum Ausbilden eines massiven Körpers oder eines Lufteinschlüsse/Porösitäten aufweisenden (porösen) Körpers miteinander verschmolzen werden. Eine gute Haltbarkeit und Belastungsakzeptanz wird neben einem schnellen Einwachsen erreicht.

Wenn der Körper eine komplette Geometrie, etwa mit Hinterschneidungen und/oder Ausnehmungen besitzt, so wird selbst das Anfertigen von patientenspezifischen Individualimplantaten möglich. Auch komplizierteste Geometrien lassen sich herstellen, wodurch ein mannigfaltiger Einsatz am beispielsweise menschlichen Körper möglich wird, insbesondere im Schädel-, Hand-, Brustbein- und Fußbereich.

Es hat sich für das Einwachsen von menschlichem Gewebe in das Implantat als vorteilhaft herausgestellt, wenn die Partikel eine kartoffelartige oder kugelartige Form besitzen.

Dabei ist es wünschenswert, wenn die Partikel in Pulverform einen Durchmesser zwischen ca. 20 µm oder ca. 50 µm und ca. 300 µm besitzen.

Die als Pulverkörner vorliegenden Partikel sollten einen Durchmesser zwischen ca. 40 µm und ca. 200 µm besitzen, vorzugsweise 140 µm.

Um jegliche Körnchen, Partikel und Pulverrestbestandteile vom Rohimplantat sowie später dem fertigen Implantat wirkungsvoll entfernen zu können, ist es von Vorteil, wenn eine Oberflächenbehandlung nach Art einer Plasmabehandlung, eines Schneestrahlens, eines druckbeaufschlagten Beschießens mit gefrorenen CO2-Flocken, etwa mittels eines druckluftgetriebenen Überschallbeaufschlagens, oder eines Ultraschallbadens durchgeführt wird.

Ein vorteilhaftes Ausführungsbeispiel ist auch dadurch gekennzeichnet, dass ein Rohimplantat einer Wärmebehandlung zur Festigkeitssteigerung unterworfen wird.

Um besonders hygienische Produkte zu erhalten, ist es von Vorteil, wenn eine Gamma-Sterilisationsbehandlung, vorzugsweise bei rund 25 kGy durchgeführt wird, beispielsweise vor dem Oberflächenbehandeln und/oder nach dem Wärmebehandeln. Als Alternative bieten sich auch Ethylenoxid (ETO)-, E-Beam-Sterilisations-, und Plasma-Sterilisationsverfahren an.

Ferner betrifft die Erfindung auch ein Implantat, hergestellt nach der erfindungsgemäßen Weise.

Ferner kann dieses Implantat auch dadurch weitergebildet sein, dass es als CMF-Implantat (Cranio-Maxillo-Facial-Implantat) zur Rekonstruktion eines Knorpel- und/oder Knochenbestandteils für einen menschlichen Körper, unter anderem als Kranialimplantat ausgebildet ist.

Es wurde durch die Erfinder gezeigt, dass es bei einer Porengröße von bis zu 600 µm schnell zum Einwachsen von Blutgefäßen und Bindegewebe kommt.

Da eine Nährstoffversorgung von vitalen Zellen innerhalb des Implantatgerüsts lediglich über eine Distanz von ca. 150 µm bis ca. 200 µm möglich ist, stellt die Neubildung von Blutgefäßen einen entscheidenden Prozess im Hinblick auf eine erfolgreiche Integration des Implantats dar. Mit dem nun vorgestellten Verfahren wird ein Einwachsen von Weichgewebe und Knochen erleichtert. Dieses umfassende vaskuläre Einwachsen hilft dabei, wichtige Zellen, die Infektionen bekämpfen, tief in das Implantat zu transportieren. Zugleich erhöht das Einwachsen von Weichgewebe die Festigkeit des Implantats. Somit wird die Nährstoffversorgung und die Festigkeit verbessert.

In der vorliegenden Erfindung werden dreidimensionale Implantate mittels eines Selective-Laser-Sintering (SLS) aus UHMWPE, HDPE und/oder PP hergestellt. Dabei werden bei einem definierten Energieeintrag die UHMWPE- und/oder HDPE- und/oder PP-Pulverpartikel ortsaufgelöst miteinander verschmolzen. Alle drei Bestandteile, nur zwei oder nur ein einziger Bestandteil wird dann (in Reinform oder in einer Mischung) miteinander / in sich verschmolzen. Mittels des erfindungsgemäßen schichtweisen Verschmelzens und anschließenden Verfestigen entsteht durch das Übereinanderlegen oder Verbinden vieler Einzelschichten ein dreidimensionales Implantat.

Es kann also eine kurzzeitige Herstellung der Implantate und eine Anpassung der Implantate an die jeweilige / geplante / gewünschte anatomische Region sichergestellt werden.

Eine Herstellung von massiven und/oder porösen geometrisch komplexen, beispielsweise patienten-spezifischen Individualimplantaten, aber auch Standardimplantaten, mittels SLS-Technologie wird möglich.

Gerade schnelle individuelle Anpassungen an Patienten werden möglich, insbesondere vor Ort, ergo am Ort der Operation.

Eine Festigkeitssteigerung wird durch eine Wärmenachbehandlung erreicht. Eine Oberflächenbehandlung ist dem Einwachsverhalten zuträglich, insbesondere wenn eine Plasmabehandlung oder eine CO₂ basierte Technologie eingesetzt wird. Die Möglichkeit der nachträglichen intra-operativen Formumgestaltung durch Wärmebehandlung wird geschaffen.

Eine mögliche Realisierung von mechanischen Verbindungsfunktionen sei erwähnt. Beispielsweise kann eine Kombination mit anderen Werkstoffen, wie Kunststoffen, etwa resorbierbaren Kunststoffen, durchgeführt werden. Eine Interkonnektierung / Verbindung, beispielsweise nach Art einer Brücke zu anderem Material oder als eine Brücke aus anderem Material ist sinnhaft realisierbar.

Die Möglichkeit der Integration von Fixierungsmöglichkeiten in Kombination mit Implantatgeometrien wird erleichtert.

Lasergesinterte poröse Implantate mit einer Gesamtporösität zwischen ca. 5% bis ca. 90 %, bezogen auf das Verhältnis vom Leervolumen zum Gesamtvolumen, werden von den Anwendern bevorzugt und mit dem vorgestellten Verfahren herstellbar. Selbst eine Gesamtporösität von über 60 % lässt sich einfach abbilden.

Es ist wünschenswert, wenn die Porengröße zwischen ca. 100 µm und ca. 3.500 µm, insbesondere ca. 80 µm bis ca. 120 µm, vorzugsweise ca. 100 µm beträgt.

Es können auch alle Schichten des Implantats aus UHMWPE und/oder HDPE und/oder PP gefertigt sein.

Alle Schichten können als poröse Schichten ausgebildet sein. Es hat sich als vorteilhaft herausgestellt, wenn das poröse, lasergesinterte Implantat in einer definierten anatomischen Region eingesetzt ist. Es kann auch eine interkonnektierende Porenstruktur erhalten werden. Ein gezieltes Aufrauen der Oberfläche auf ca. 5 µm bis ca. 900 µm ist denkbar. Das poröse, lasergesinterte Implantat enthält jedoch vor dem Einsatz keinerlei Restpulverpartikel mehr. Die Wärmebehandlung wird derart durchgeführt, dass kein Verschließen der Poren stattfindet. Es wird eine Festigkeitssteigerung zwischen den interkonnektierenden Porensträngen erreicht. Eine Oberflächenbearbeitung mittels Heißluft, Infrarotstrahlern und/oder thermisches Entgraten und/oder Explosionsentgraten findet statt. Ein Verschmelzen/Versiegeln ohne Porenverschluss ist die Folge. Dabei kann Sauerstoff und Brennstoff sowie ein optionales Zusatzmittel bei ca. 3.000 °C gezündet werden.

Alternativ ist auch eine Wärmebehandlung unter Einsatz von Heißluft praktikabel. Hier bewährt sich der Einsatz eines Heißluftstroms mit einer Temperatur von 300°C bis 650°C. Die Temperatur am Implantat ist während der Behandlung allerdings geringer. Als Abstand sollte ca. 10 cm bis 30 cm eingehalten werden. Die Wärmebehandlung wird für ca. 5 Sekunden bis 60 Sekunden durchgeführt. Dabei wird eine Reduzierdüse mit einem Durchmesser von 14 mm auf 9 mm, oder eine Breitstrahldüse von 50 mm mal 2 mm bis 5 mm bzw. 75 mm mal 2 mm bis 5 mm, oder eine Breitschlitzdüse eingesetzt.

Es ist von Vorteil, wenn das Implantat hydrophob und/oder hydrophil ist. So kann eine Seite hydrophob sein und die andere Seite hydrophil. Das Grundmaterial kann beispielsweise hydrophob sein. Bei Behandlungen mit Niederdruckplasma wird eine optimale Struktur erreicht. Die Beschichtung kann beispielsweise so aufgebracht werden, dass ein hydrophiles Verhalten in einem bestimmten Bereich, beispielsweise nur auf einer Seite vorliegt. Dadurch kann ein schnelleres Einwachsen von dieser Seite erreicht werden. Das Implantat kann mit Niederdruckplasma behandelt werden.

Hat das Implantat daher grundsätzlich die eine Eigenschaft, bspw. hydrophob, kann die andere Eigenschaft mittels einer Beschichtung hervorgerufen werden, beispielsweise die hydrophile Eigenschaft. Dies ist auch vice versa möglich.

Die besagten Partikel der Gruppe aus UHMWPE, HDPE und/oder PP können auch ausschließlich und oder zumindest maßgeblich / überwiegend verwendet werden. Mischungen exklusiv daraus sind insbesondere möglich.

## Patentansprüche

1. Verfahren zum Herstellen eines Implantats, wobei Partikel der Gruppe aus Ultra-high-molecular-weight Polyethylene (UHMWPE) und/oder High-density Polyethylene (HDPE) und/oder Polyprophylen (PP) mittels eines Selective Laser Sintering-Verfahrens schichtweise miteinander verschmolzen werden, **dadurch gekennzeichnet, dass** eine Wärmebehandlung nach dem Selective Lasersintern so durchgeführt wird, dass Poren des zu schaffenden Implantats zu einer Oberfläche des Implantats unverschlossen bleiben oder eine Wärmebehandlung nach dem Selective Lasersintern so durchgeführt wird, dass die Poren des zu schaffenden Implantats zu einer Oberfläche des Implantats nur in Teilbereichen geschlossen werden.

2. Verfahren nach Anspruch 1, **dadurch gekennzeichnet, dass** die Partikel zum Ausbilden eines massiven Körpers oder eines Lufteinschlüsse aufweisenden porösen Körpers miteinander verschmolzen werden.

3. Verfahren nach Anspruch 2, **dadurch gekennzeichnet, dass** der Körper eine komplexe Geometrie besitzt.

4. Verfahren nach einem der Ansprüche 1 bis 3, **dadurch gekennzeichnet, dass** die Partikel eine kartoffelartige oder kugelartige Form besitzen.

5. Verfahren nach Anspruch 4, **dadurch gekennzeichnet, dass** die Partikel in Pulverform einen Durchmesser zwischen ca. 20 µm und ca. 300 µm besitzen.

6. Verfahren nach Anspruch 5, **dadurch gekennzeichnet, dass** die als Pulverkörner vorliegenden Partikel einen Durchmesser zwischen ca. 130 µm und ca. 150 µm besitzen.

7. Verfahren nach einem der Ansprüche 1 bis 6, **dadurch gekennzeichnet, dass** eine Oberflächenbehandlung nach Art einer Plasmabehandlung, eines Schneestrahlens, eines druckbeaufschlagten Beschießens mit gefrorenen CO₂-Flocken oder eines Ultraschallbades durchgeführt wird.

8. Implantat, hergestellt nach dem Verfahren gemäß einem der vorhergehenden Ansprüche.

9. Implantat nach Anspruch 8, **dadurch gekennzeichnet, dass** es als CMF-Implantat zur Rekonstruktion eines Knorpel- und/oder Knochenbestandteils für einen menschlichen Körper ausgebildet ist.

## Claims

1. A method for producing an implant, wherein particles of the group of ultra-high molecular weight polyethylene (UHMWPE) and/or high-density polyethylene (HDPE) and/or polypropylene (PP) are fused together layer by layer by means of a selective laser sintering method, **characterized in that** a heat treatment is carried out after the selective laser sintering such that pores of the implant to be produced remain unsealed to a surface of the implant or a heat treatment is carried out after the selective laser sintering such that the pores of the implant to be produced are sealed to a surface of the implant in partial areas only.

2. The method according to claim 1, **characterized in that** the particles are fused together for forming a massive body or a porous body including entrapped air.

3. The method according to claim 2, **characterized in that** the body has a complex geometry.

4. The method according to one of the claims 1 to 3, **characterized in that** the particles have a potato-like or sphere-like shape.

5. The method according to claim 4, **characterized in that** the particles in powder form have a diameter between about 20 µm and about 300 µm.

6. The method according to claim 5, **characterized in that** the particles present as powder grains have a diameter between about 130 µm and about 150 µm.

7. The method according to one of the claims 1 to 6, **characterized in that** a surface treatment is carried out in the form of a plasma treatment, a snow blasting, a pressurized bombarding by frozen flakes or an ultrasonic bath.

8. An implant produced according to the method according to one of the preceding claims.

9. The implant according to claim 8, **characterized in that** it is formed as a CMF implant for reconstruction of a cartilage and/or bone component for a human body.

## Revendications

1. Procédé de fabrication d'un implant, dans lequel des particules du groupe constitué de polyéthylène de masse moléculaire très élevée (UHMWPE) et/ou de polyéthylène haute densité (HDPE) et/ou de polypropylène (PP) sont fusionnées entre elles par couches au moyen d'un procédé de frittage sélectif au laser, **caractérisé en ce qu'**un traitement thermique après le frittage sélectif au laser est effectué de sorte que des pores de l'implant à créer restent non fermés par rapport à une surface de l'implant ou qu'un traitement thermique après le frittage sélectif au laser est effectué de sorte que les pores de l'implant à créer ne sont fermés que dans des zones partielles par rapport à une surface de l'implant.

2. Procédé selon la revendication 1, **caractérisé en ce que** les particules sont fusionnées entre elles pour former un corps massif ou un corps poreux présentant des poches d'air.

3. Procédé selon la revendication 2, **caractérisé en ce que** le corps comporte une géométrie complexe.

4. Procédé selon l'une quelconque des revendications 1 à 3, **caractérisé en ce que** les particules ont une forme de pomme de terre ou de sphère.

5. Procédé selon la revendication 4, **caractérisé en ce que** les particules sous forme de poudre ont un diamètre compris entre env. 20 µm et env. 300 µm.

6. Procédé selon la revendication 5, **caractérisé en ce que** les particules se présentant sous forme de grains de poudre ont un diamètre compris entre env. 130 µm et env. 150 µm.

7. Procédé selon l'une quelconque des revendications 1 à 6, **caractérisé en ce qu'**un traitement de surface de type traitement par plasma, par projection de neige, par bombardement sous pression de flocons de CO2 congelés ou par bain à ultrasons est effectué.

8. Implant fabriqué par le procédé selon l'une quelconque des revendications précédentes.

9. Implant selon la revendication 8, **caractérisé en ce qu'**il est conçu en tant qu'implant CMF pour la reconstruction d'un composant cartilagineux et/ou osseux pour un corps humain.
